# EUROPEAN PATENT SPECIFICATION

(11) **EP 1 582 869 B1**
(45) Date of publication and mention of the grant of the patent: **31.12.2008**
(21) Application number: 05001804.3
(22) Date of filing: 28.01.2005
(51) Int. Cl.: G01N 33/558, G01N 33/543

(54) **Interrupted flow testing device and method for immunological confirmatory tests**
Unterbrochene Durchfluss-Testvorrichtung und Methode für immunologische Bestätigungstests
Dispositif d'écoulement interrompue et procédé comme test de confirmation immunologique

(30) Priority: 28.01.2004 US 767897
(43) Date of publication of application: 05.10.2005
(73) Proprietor: DNT Scientific Research, LLC, San Diego, CA 92129 (US)
(72) Inventor: Naishu Wang, Poway, CA 92064 (US); David F. Zhou, Poway, CA 92064 (US)
(74) Representative: Fox-Male, Nicholas Vincent Humbert

(56) References cited:
- WO-A-94/23300
- WO-A-99/46591

## Description

### Field of the Invention

This invention relates to rapid confirmatory testing devices for analyzing body fluids and other fluids using an immunochromatography, and more particularly to apparatuses for detection of antibodies, and/or antigens in a point-of-care fluid test.

### Background of the Invention

Over past decades, the prior art has offered several types of rapid diagnostic testing techniques primarily for body fluids. First, were the latex particle agglutination tests, then the Flow Through tests leading to the current Lateral Flow Single Step test. To this day, the Western Blot Analytical Assay is the only one reliably used for the confirmatory detection of HIV infection in a clinical laboratory setting. Due to its multi-step manipulation and verification phases, completion of this type of assay takes days, if not weeks. Such a delay can unfortunately lead to further propagation of infectious pathogens such as HIV or other serious results, such as the metastasis of cancers. There is no practical or economical confirmatory rapid diagnostic testing technique for use in a point-of-care setting, available in the market place today.

The instant invention results from an attempt to refine the accuracy and expedite the performances of the common chromatographic rapid testing devices to a higher and new level for use in early confirmatory and speedy detection of the presence of pathogens or pathogenic conditions such as occurs with HIV infection, cancers and other disorder. Such a device could be easily adapted to detect certain lethal viruses, bacteria or other pathogenic antigens/antibodies in body fluids and other fluids used also in certain food and environmental testing, and to detect other abnormalities in body fluids which are indicative of various cancers, and cardio-vascular diseases, and/or other diseases.

### Summary Of The invention

The present invention provides a device as in Claim 1.

The device may include the features of any one or more of dependent Claims 2 to 8.

The principal and secondary objectives of the invention may be to provide a device to more rapidly conduct a confirmatory immunoassay test a point-of-care setting.

These and other valuable objects are achieved by a self-contained, multi-stage, programmed, interrupted downward flow, rapid confirmatory immunological testing ("RCIT") apparatus contained in a single molded enclosure. The apparatus carries a number of chromatographic test strips in an inclined orientation forming down-flow strips. The primary exposure of a volume-measured fluid specimen to a pathogenically specific antibody and/or antigen (or to a group thereof) in a colloidal gold, or other type conjugate (for example, colloidal carbon, latex beads, or magnetic beads) in a volume measured, reactive, buffered solution occurs in a first chamber before flowing on to contact the down-flow strips in a second chamber. A holding reservoir located between the first and the second chambers temporarily restricts the flow of the mixture to allow a short period of incubation before proceeding with the secondary specific immunological binding reaction in the down-flow strips. The down-flow strips preferably include a single layer of uniformly dispersed porous matrix material such as polyethylene commercially available from Porex Corporation of Fairburn, Georgia coated with a number of corresponding antigenic epitopes which are immune-determinant of the pathogen such as the HIV or the condition such as cancer. According to standard Western Blot assay procedure, in HIV confirmatory detection, the appearance of at least two epitope lines on a down-flow strip not only confirms the presence of HIV, but also gives an analytical indication of the type of antibodies present in the specimen which can change during different periods of HIV infection and Acquired Immune Deficiency Syndrom (AIDS). Additionally the down-flow strip or strips include a control line, working as an internal system control indicator. Therefore, any positive result shown by this RCIT method will include at least three (3) lines appearing in the test reaction window.

In one embodiment, a supply of aqueous buffer solution is held in a sealed tank until the sample specimen has been introduced into the device and the cap has been closed. A prong in the undersurface of the cap punctures a membrane sealing the upper opening of the tank allowing the buffer solution to be dispensed into the first chamber under atmospheric pressure. A pad at the bottom of the second chamber in contact with a lower part of the strips absorbs the excess wash buffer that has not been retained by the strip. The strips can be positioned in an incline rather than straight vertical position in order to reduce the height of the device. The flow out of the incubation reservoir and into the strip is prompted by a combination of siphoning, gravity and capillarity action forces.

In another embodiment of the apparatus the enclosure has a slip-cover which is retracted to extend a spring-loaded support leg for orienting the device on an incline from horizontal. A first measured supply of aqueous mix buffer solution preferably containing the appropriate gold or other type conjugate in suspension is held in a sealed tank until the sample specimen has been introduced through an inlet into the measured first chamber. Under the push of a manipulable member, the tank seal is then broken, allowing the buffer to mix with the sample to form a mixture which then flows out of the first chamber, through a filter into an incubation reservoir into which extends the ends of one or more down-flow oriented test strips.

The flow out of the incubation reservoir to the non-horizontal strips is prompted by a combination of siphoning, gravity and capillarity action forces. A hydrophilic absorptive pad such as a synthetic sponge is placed at the bottom of the second chamber in contact with a lower part of the strips to absorb the fluid and encourage the siphoning action. After waiting for an adequate amount of the mixture to flow down into and through the strips, a bladder containing an amount of wash buffer solution is opened under the push of another manipulable member to wash down the remaining mixture through the strips and end the reaction at the epitope lines on the strips. The lower pad absorbs the wash buffer and, along with the angled orientation of the strips, discourages reverse flow of fluid back up into the strips. Both of the manipulable members are slidable, built-in components of the apparatus.

Apart from its unique, self-contained measured volume mix and wash buffer tanks, and its simplified program for successively opening these tanks during processing, it is the interrupted down-flow action caused by siphoning, gravity, and to a lesser extent capillarity, from the incubation reservoir that improves accuracy and makes the apparatus well suited to rapid diagnostic point-of-care testing. The apparatus' ability to detect multiple antigenic markers or specific antibodies for single or multiple pathogens associated with one or more diseases or conditions provides a panel or profile based diagnosis with a high degree of accuracy (including the high sensitivity and specificity with a rapid speed to show the results and a long term of stability for room temperature storage), closely approach or even surpassing the accuracy of Western Blot Analysis.

### Brief Description of the Drawings

**Fig. 1** is a diagrammatical cross-sectional illustration of a first embodiment of the interrupted flow testing device according to the invention.
**Fig. 2** is a perspective view of the device of Fig. 1.
**Fig. 3** is a perspective view of a second embodiment of the interrupted down-flow testing apparatus according to the invention in its retracted position.
**Fig. 4** is a perspective view of the interrupted down-flow testing apparatus of Fig. 3 in its extended position.
**Fig. 5** is a diagrammatical cross-sectional side view of the device of Fig. 4 taken along line 5-5.
**Fig. 6** is a diagrammatical partial perspective view of the premix vessel portion of the apparatus indicating the orientation of cross-section planes for Figs. 7, 10 and 11 (not claimed).
**Fig. 7** is a diagrammatical cross-sectional side view of a first embodiment of the premix vessel (not claimed).
**Fig. 8** is a diagrammatical partial perspective view of the wash bladder and puncturing prongs portion of the apparatus in an extended position (not claimed).
**Fig. 9** is a diagrammatical cross-sectional side view of the rear portion of the enclosure showing the wash buffer bladder and puncturing prongs in their pretest position (not claimed).
**Fig. 10** is a diagrammatical cross-sectional side view of an alternate embodiment of the premix vessel (not claimed).
**Fig. 11** is a diagrammatical cross-sectional top view of another alternate embodiment of the premix vessel (not claimed).

### Description of the Preferred Embodiment

The preferred embodiments will be described in connection with the detection of HIV in a fluid specimen. Those skilled in the art will readily appreciate adaptation of these embodiments to detect other pathogens, or pathogenic conditions within body, food or environmental fluid samples.

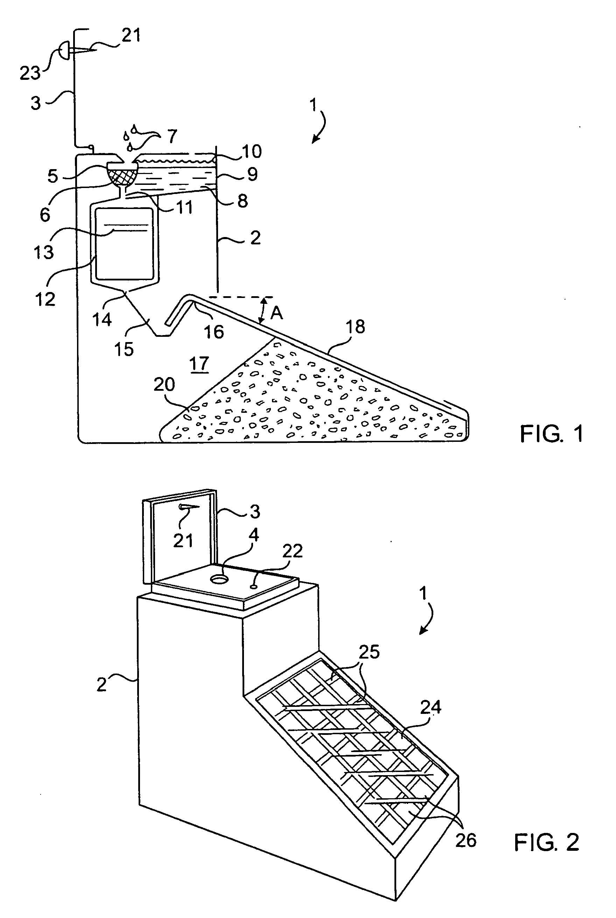

Referring now to the drawing, there is shown in Fig 1-2 a first embodiment of an immunoassay testing device **1** according to the invention. The device is preferably packaged in a molded plastic enclosure **2** topped by a sealing cap **3**. In the upper region of the device, and immediately under a ceiling hole **4** is a sampling well **5**. The internal wall of the well is funnel-shaped, and retains some filtration material **6**. The geometry of that wall, whether in the form of a V or a U, has a portion of a relatively low pitch so that when a fluid specimen **7** such as whole blood, **7** or saliva runs along the wall, particles and adhesive matters are separated from the fluid component of the specimen. A supply of aqueous buffering solution **8** is held in a tank **9** along side the sampling well. The tank has a top opening hermetically sealed by a membrane **10,** and a dispensing port **11** in a lower region leading to a first chamber **12** in a first analytical part of the device. The chamber is located immediately below the sampling well and receives the fluid component and is subjectable to the buffering solution. That first chamber holds colloidal gold conjugated specific to the HIV antigen or other colloidal conjugate **13** specific to the condition being tested that reacts with the fluid specimen in its buffered solution.

An outlet **14** at the bottom of the chamber leads to an incubation reservoir **15** in which the solution flowing from the chamber accumulates and rests until the level of the solution reaches an upper part of the reservoir where an escape port **16** leads to a second chamber **17** holding one or more chromatographic testing strips held in a non-horizontal, downward flow orientation **18** and can be referred to as down-flow strips. The membrane portion of the down-flow strips is preferably a single layer of uniformly dispersed porous matrix material such as polyethylene commercially available from Porex Corporation of Fairburn, Georgia. Each down-flow strip **18** is preferably positioned in an inclined position at a pitch angle A of at least 15 degrees from the horizontal. The upper edge **19** of the strip dips into the reservoir and is contacted by the solution that flows down slowly under the effect of capillarity, gravity and siphoning forces enhanced by an absorbing pad **20** positioned in the bottom of the enclosure and in contact with the lower portion of the strip **18**.

The down-flow strip is coated with a number of epitopes that are immune-determinant of the HIV virus such as p18, p24, p32, gp36, gp41, p51, p55, p65, gp120, gp160 and subtype o, etc.

It should be noted that the escape port **16** acts as a means for restricting the flow therethrough. Further, is should be understood that the dispensing of the buffer solution **8** out of the tank **9** is triggered by puncturing the membrane **10**. The puncturing is accomplished by a prong **21** which extends from the underside of the cap **3** and passes through an aperture **22** in the roof of the enclosure. The prong is normally held into a retracted position during storage and shipment of the device, but can be moved to an extended position by manipulating a knob **23** on the outside of the cap. The prong is positioned, shaped and dimensioned to extend sufficiently through the aperture **22** into rupturing contact with the membrane **10**.

The buffer solution **8** washes and carries the components of the specimen that comes down from the sampling well and provide the volume of fluid necessary **15** to fill the incubation reservoir and thus, regulate the transfer of the specimen through the device. By adjusting the volume of buffer solution to what is necessary to create a minor overflow of the reservoir, excessive flooding of the test strip is avoided.

It should be understood that the flow-interrupting reservoir **15** can assume a variety of positions and configurations that provides a temporary, but longer incubation time for the buffered sampling solution to complete the first affinity binding of the immunno-chemical reaction before it is contacted with the chromatographic testing strip to form the second affinity binding of the Immuno-Chemical reaction, forming the double antigen (or double-antibody in some cases) sandwich-immuno complex. A transparent window **24** sealed to the enclosure provides a direct viewing of chromatographic test lines **25** appearing on a number of down-flow test strips **26**.

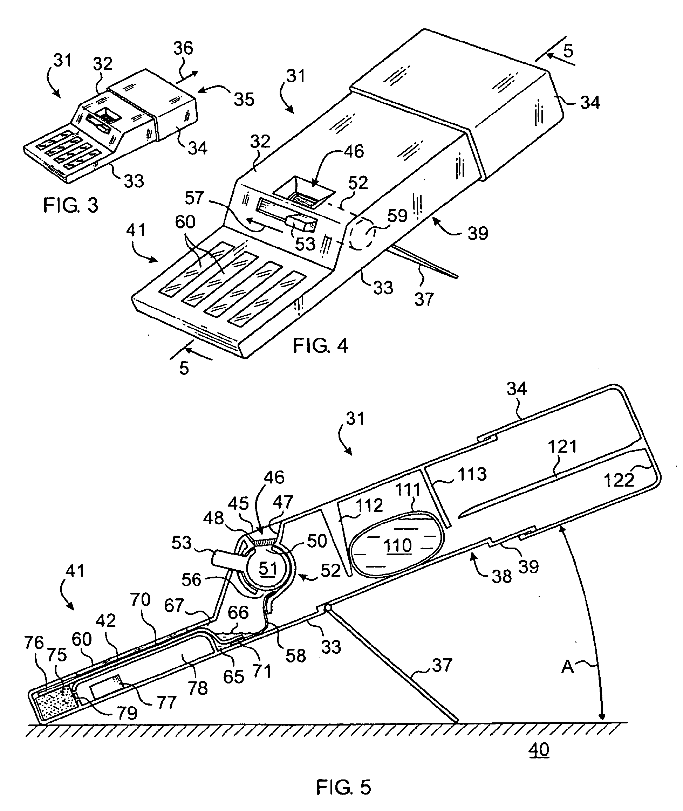

Referring now to Figs. 3-5, there is shown a second embodiment of an immunoassay testing apparatus **31** according to the invention. The apparatus is preferably packaged in a molded plastic enclosure **32** having a base pan **33**. An extendable and retractable slip-cover **34** is slidingly mounted at the back end **35** of the enclosure. The slip-cover is in a retracted position (as shown in Fig.3) during storage and shipment of the device, but can be slid rearwardly to an extended position (as shown in Fig.4) by pulling axially backward **36** on the slip-cover while holding the base pan **33** stationary. This action also causes the extension of a spring-loaded support leg **37** hingedly mounted within a recess **38** in the bottom outer surface **39** of the enclosure. The leg supports the apparatus upon a level surface **40** so that a test station **41** holding one or more down-flow testing strips **42** is oriented in an inclined position at a pitch angle **A** of at least 15 degrees from the horizontal.

In the medial region of the apparatus, and immediately under a ceiling hole **45** is a sampling well **46.** The internal wall **47** of the well is funnel-shaped. The geometry of that wall, whether in the form of a V or a U, has a portion of a relatively low pitch so that when a fluid specimen such as whole blood or saliva runs along the wall, particles and adhesive matters are separated from the fluid component of the specimen. Alternately, or in addition to this feature, a screen **48** can be placed in the well to further help separate coarse particulates. The sampling well **46** leads downwardly through an inlet **50** to a first chamber **51** of a premix vessel **52** which receives the fluid component of the specimen in a first analytical part of the apparatus.

Upon sliding manipulation **57** of a knob **53** the premix vessel exposes and premixes the specimen with a measured amount of mix buffer solution **59** containing the colloidal gold conjugate specific to the HIV antigen and carried in an openable tank withing the premix vessel. The first chamber is therefore subjectable to the mix buffer solution. In this way, the knob and vessel form a built-in manipulable member for releasing the mix buffer solution onto the specimen.

The amount of mix buffer solution is selected to adequately react with the amount of sample, and in this embodiment is preferably between about 200 and 300 microliters, and most preferably about 250 microliters. The mixture is dispensed through an outlet **56** at the bottom of the vessel through a sheet of filter material **58** such as fibreglass (or 3M paper etc.) to further screen out finer particulates before flowing on to one or more down-flow testing strips **42** held in the test station **41**. A transparent window **60** sealed to the enclosure provides a direct viewing window of down-flow test lines appearing on the strips **42**.

The mix buffer solution **59** preferably holds in suspension an amount of colloidal gold conjugated specific to the HIV antigen for reaction with the specimen. In this way the solution can be characterized as a reactive solution. Alternately, or additionally, the filter material 58 can be impregnated with the proper gold conjugate to further react with the mixture flowing therethrough.

Situated between the premix vessel **52** and the test station **41** is an incubation reservoir **65** into which the mixture briefly accumulates and rests before being drawn into a second chamber **70** housing the strips. In the angled, test orientation, the reservoir has a given capacity. The location and capacity of the reservoir interrupts the flow, giving the mixture more time for the first affinity binding of the immunno-chemical reaction to occur and reach a maximum, before the mixture is contacted with the down-flow strips to form the second affinity binding of the immuno-chemical reaction. Within about 0.5-2 minutes, the relative slow speed of the downward flow of the mixture caused by the combination of siphoning, gravity and capillarity action forces, promotes a maximum degree of the second affinity binding on the down-flow strips. The two maximized affinity binding steps in turn maximize the diagnostic sensitivity of the RCIT device technology.

The upper edge **71** of the strips dip into the reservoir **65** and are contacted by the pooled mixture **66**. The mixture flows into the strips, under the effect of siphoning, gravity and capillarity forces, over the upper part of the reservoir through an escape port **67** and into the second chamber **70**. Flow through the strips is enhanced by an absorbing pad **75** positioned in the bottom front end of the enclosure and in contact with the undersurface **76** of a lower portion of the strips **42**. The size of the pad is selected to accommodate the combined volume of the fluids within the apparatus. A block of desiccant **77** is held in a third chamber **78** in the enclosure and is in communication with the second chamber **70** through holes **79** to help provide a room temperature storage shelf life of at least **24** months. Additionally, the entire apparatus is preferably kept in a sealed plastic foil pouch bag until use.

It should be understood that the flow-interrupting reservoir **65** can assume a variety of positions and configurations that provide a temporary, but longer incubation time for the mixture to complete the first affinity binding.

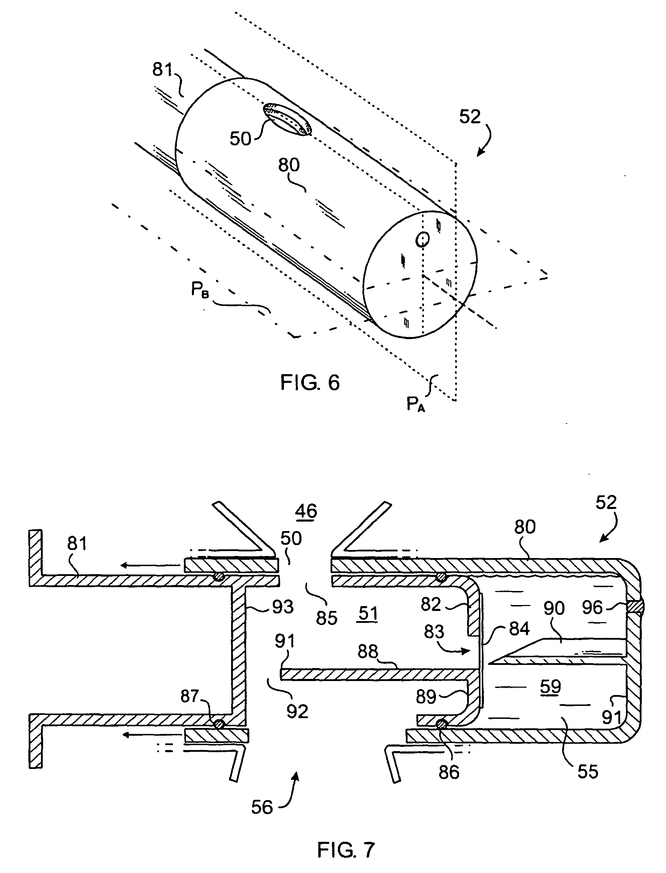

Referring now to Figs 5-7, the vessel **52** is formed to have a substantially cylindrical and axially translatable receptacle **80** engaged by substantially stationary piston **81**. The head **82** of the piston and the receptacle define an enclosed and movable tank **55** which carries a measured supply of an aqueous mix buffer solution **59.** The piston is hollow to define an internal cavity forming the first premix chamber **51.** The head of the piston is interposed between and separates the tank from the premix chamber residing adjacent to it. A passageway **83** is formed through the piston head and is initially hermetically sealed by a frangible membrane 84 preferably made from a plastic laminated foil heat sealed to the outer surface of the piston head.

The fluid specimen, which is typically a drop of blood for HIV testing, enters the first, premix chamber **51** through an intake aperture **85** initially positioned below the vessel inlet **50** cut through the receptacle **80** and comes to rest upon a substantially horizontal shelf **88** extending from the inner wall **89** of the piston head toward a medial position in the premix chamber below the intake aperture **85** and terminating at an end **91** to form a drain **92** between the shelf end and the back wall **93** of the chamber. The viscosity of the blood prevents it from flowing on its own through the drain. The location and dimensions of the shelf and drain can be adjusted or further selected to adapt the apparatus to other different viscosity fluid specimens.

The test is initiated by sliding manipulation of the knob **53** which is connected to the receptacle **80**. The sliding translation of the receptacle over the piston 81, causes an axially channeled spike **90** extending from an inner wall 91 of the tank to puncture the membrane **84** allowing the mix buffer solution **59** to be forcefully dispensed through the passageway **83** into the first chamber **51.** Further translation volumetrically compresses the tank **55.** The dimensions of the passageway, the spike and the spike's channel can be selected to cause a jet of buffer fluid to be directed against the specimen causing an agitated mixing of the buffer with the specimen to form a more thorough mixture. The lower viscosity mixture is then able to flow through the drain **92** and out of the vessel. Translation moves the position of the vessel inlet **50** to close the intake aperture **85** preventing backward flow of specimen out of the aperture. O-rings **86,87** discourage flow of fluid in the gap between the receptacle and piston.

It should be noted that the dispensing of the mixture out of the vessel **52** is triggered by puncturing the membrane **84**. A plug **96** seals a tank fill port through the end wall of the receptacle.

Referring to Figs 5, 8 and 9, the apparatus also provides for the supply of a stop wash buffer solution to the down-flow strips in order to stop the reaction in the strips and to carry away lingering chemicals and residue which could serve to obscure the lines formed on the strips and also to remove any other non-specific materials from the reaction area. The wash buffer is preferably applied after a certain programmed waiting period which allows for the mixture to be drawn through the strips to an adequate degree. The preferred waiting time is of course dependent on the type of test being performed. For HIV detection the waiting period is preferably between about 0.5 and 2 minutes. The amount of wash buffer solution is selected to adequately wash the down-flow strips without unduly increasing the bulk of the apparatus, and in this embodiment is preferably between about 2 and 3 milliliters, and most preferably about 2.5 milliliters. In this embodiment the volume of wash buffer is about ten times that of the mix buffer.

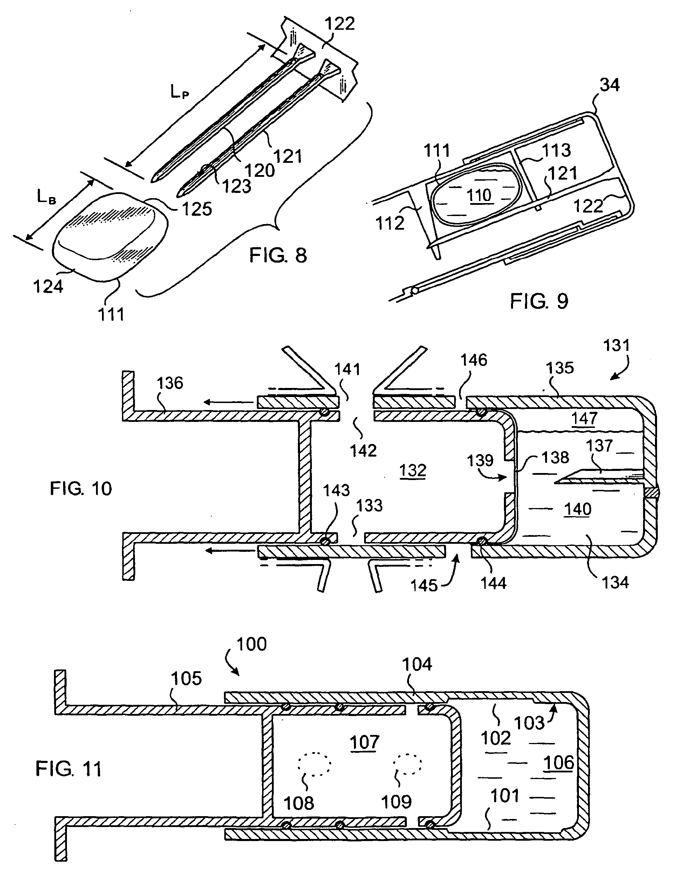

The stop wash buffer solution **110** is contained in an frangible second tank or bladder **111** located upstream from the reservoir **65**. In its pretest position (as shown in Fig. 9) the bladder rests atop a pair of prongs **120,121** extending forward from the rear wall **122** of the slip-cover **34** between a front stop **112** and a back stop **113** extending down from a ceiling of the enclosure. When the slip-cover **34** is moved to its extended position (as shown in Fig. 5 and 8) the bladder falls to rest on the base pan **33**. The bladder is opened by re-engaging the slip cover **34** which causes the prongs **120,121** to puncture the bladder. It is understood that the bladder is in communication with the test station **41** meaning there is a fluid path from the bladder through the reservoir to the station. In this way, the slip-cover and prongs form a built-in, manipulable member for releasing the wash solution onto the down-flow strips.

As shown in Fig. 8, each prong is shaped to have an axial groove **123** which acts as an air channel to encourage the evacuation of the bladder when it is punctured. Further, the axial length **L_{P}** of the prongs is selected to be greater than the length **L_{B}** between the forward and rear axially opposite walls **124,125** of the bladder so that in the re-engaged position the prongs puncture both axially opposite walls causing the wash buffer to flow more rapidly out of the bladder which ensures rapid washing of the down-flow strips at the preselected appropriate time.

The second down-flow of the said stop wash buffer occurs relatively faster than the first down-flow of the mixture. The wash buffer having about ten times the volume of the mixture flushes out the non-specific binding caused by non-specific materials in the reaction area. This action maximizes the specificity of the present device to provide RCIT.

Referring now to Fig. 10, there is shown an alternate embodiment of the premix vessel **131** particularly adapted to samples having a relatively low viscosity such as urine. This embodiment is characterized by its premix chamber **132** having an initially sealed, openable drain **133**. Further, the tank **134** formed between the receptacle **135** and the piston **136** is made to be volumetrically compressible prior to the spike **137** puncturing the membrane **138** sealing the passageway **139**. In this way the mix buffer solution **140** is pressurized prior to puncturing to cause vigorous, thorough mixing. Further, the premix chamber is formed to have a limited volume which acts to measure the amount of the fluid specimen.

As the receptacle **135** is translated onto the piston **136**, the receptacle acts as a movable carriage for the mix buffer tank and volumetrically compresses the tank **134**. Further translation moves the position of the inlet **142** to close the intake aperture **141** preventing backward flow of specimen out of the inlet. O-rings **143,144** discourage flow of fluid in the gap between the receptacle and piston. Further translation causes the spike **137** to puncture the membrane **138** allowing the mix buffer solution to be forcefully dispensed through the passageway **139** into the first chamber **132** under pressure, collapsing the tank and thoroughly mixing the buffer with the specimen to form a mixture. Once fully collapsed, an outflow aperture **145** aligns with the vessel outlet **133** to allow evacuation the mixture therethrough. Full translation also causes the inlet **142** to be positioned below an air vent **146** which facilitates downward flow of the mixture out of the premix chamber by discouraging the formation of a vacuum.

It should be noted that the dispensing of the mixture out of the vessel **131** is triggered by puncturing the membrane **138**. Further, in addition to the mix buffer supply, the tank holds an amount of a gas **147** to facilitate the volumetric compression of the tank during translation of the receptacle but before the membrane is punctured. The gas can be air, or is more preferably a gas which is inert or less reactive with the mix buffer and its suspended conjugates such as nitrogen in an effort to help the suspended conjugates to have a longer stability and shelf-life at room temperature.

Referring now to Fig. 11, there is shown a second alternate embodiment of the premix vessel **100** in which the passageway is formed by axial depressions or channels **101,102** set into the inner surface **103** of the receptacle **104**. In this way, translation of the piston **105** beyond a given distance will place the channels in communication with both the tank **106** and premix chamber **107** allowing the pressurized flow of the mix buffer into the first, premix chamber to mix with the specimen deposited through an inlet **108** and dispensed through a drain **109.**

The interrupted down-flow test can rapidly provide an analytical panel or profile of antigen or antibody detection, and confirm the biochemical or pathogenic condition such as HIV infection, or early stage cancer prior to metastasis, or acute cardiac disorder by way of a simple, inexpensive and disposable device that can be manipulated safely by a relatively low skilled person. The above apparatus also benefits from dry chemistry storage, for an economically prolonged room temperature shelf life of at least 24 months.

It should be noted that the down-flow strip can be adapted especially for a downward movement of two interrupted but consecutive (with only a minute apart from each other) flows utilizing a combination of siphoning, gravity, and to a lesser extent capillarity. Because the first affinity binding has already occurred in the first flow reaching the strip, the strip does not need to be directly connected with any colloidal conjugate pad, but only links with the incubation reservoir at its top end. Further, its membrane portion can be made from a single layer of uniformly dispersed porous matrix material such as polyethylene.

The quality of the clinical performance of this novel platform technology surpasses any previous rapid testing technologies, such as Latex particle agglutination, Flow-Through test, and the currently wide-spread Lateral Flow devices. It is a technology of Rapid Confirmatory Immunological Testing (RCIT).

## Claims

1. A flow immunoassay testing device which comprises:
a first chamber subjectable to a supply of buffer solution;
a second chamber holding at least one chromatographic testing strip and an absorbing pad; and
a flow-interrupting reservoir of a given capacity between said first and second chambers;
said reservoir having an escape port in an upper part thereof;
said strip having an upper edge, in use, dipping into said reservoir through said escape port and a downward pitch between said port and a lower portion in contact with said pad;
whereby, in use, a liquid accumulated in said reservoir can flows into said strip under the effect of capillarity, gravity and siphoning forces.

2. A testing device according to Claim 1, **characterized in that** said strip is held in a non-horizontal orientation.

3. A testing device according to one of the preceding claims, **characterized in that** said strip comprises a single layer of uniformly dispersed porous matrix material.

4. A testing device according to one of the preceding claims, **characterized in that** said solution comprises a conjugate specific to a condition being tested.

5. A testing device according to one of the preceding claims, which further comprises means for triggering the dispensing of said supply into said first chamber.

6. A testing device according to one of the preceding claims, which further comprises a sampling well in communication with said first chamber and having a funnel-shaped internal wall.

7. A testing device according to one of the preceding claims, which further comprises an absorbing pad in contact with a portion of said strip.

8. A testing device according to one of the preceding claims, **characterized in that** said supply of buffer solution is adjusted to create a minor overflow of said reservoir.

## Patentansprüche

1. Flow-Immunassay-Testvorrichtung, umfassend:
eine erste Kammer, die einer Zufuhr von Pufferlösung ausgesetzt sein kann;
eine zweite Kammer, die mindestens einen chromatographischen Teststreifen und ein absorbierendes Kissen enthält; und
ein Fluss unterbrechendes Reservoir einer gegebenen Kapazität zwischen der ersten und der zweiten Kammer;
wobei das Reservoir eine Ausströmöffnung in einem oberen Teil desselben hat;
wobei der Streifen eine Oberkante hat, die beim Einsatz in das Reservoir durch die Ausströmöffnung eintaucht, und eine nach unten gerichtete Abschrägung zwischen der Öffnung und einem unteren Abschnitt in Kontakt mit dem Kissen hat,
wodurch beim Einsatz eine Flüssigkeit, die sich im Reservoir angesammelt hat, unter dem Einfluss der Kapillarität, Schwerkraft und Abzugskräfte in den Streifen fließen kann.

2. Testvorrichtung nach Anspruch 1, **dadurch gekennzeichnet, dass** der Streifen in einer nicht-horizontalen Lage gehalten ist.

3. Testvorrichtung nach einem der vorherigen Ansprüche, **dadurch gekennzeichnet, dass** der Streifen eine einzige Schicht aus gleichförmig verteiltem porösem Matrixmaterial umfasst.

4. Testvorrichtung nach einem der vorherigen Ansprüche, **dadurch gekennzeichnet, dass** die Lösung ein Konjugat umfasst, das spezifisch für einen Zustand ist, der gerade geprüft ist.

5. Testvorrichtung nach einem der vorherigen Ansprüche, die ferner Mittel zum Auslösen der Abgabe des Zufuhrmittels in die erste Kammer umfasst.

6. Vorrichtung nach einem der vorhergehenden Ansprüche, die ferner einen Probennahme-Well umfasst, der in Verbindung mit der ersten Kammer steht und eine trichterförmige Innenwand hat.

7. Testvorrichtung nach einem der vorherigen Ansprüche, die ferner ein absorbierendes Kissen in Kontakt mit einem Teil des Streifens umfasst.

8. Testvorrichtung nach einem der vorherigen Ansprüche, **dadurch gekennzeichnet, dass** die Zufuhr von Pufferlösung so eingestellt ist, dass sie einen geringen Überlauf aus dem Reservoir erzeugt.

## Revendications

1. Dispositif de test d'un essai immunologique à écoulement qui comprend :
une première chambre pouvant être soumise à un apport de solution de tampon ;
une deuxième chambre soutenant au moins une bande de test chromatographique et un tampon absorbant ; et
un réservoir interrompant l'écoulement d'une capacité donnée entre lesdites première et deuxième chambres ;
ledit réservoir ayant un orifice d'échappement dans une partie supérieure de celui-ci ;
ladite bande ayant une bordure supérieure trempant, lors de l'utilisation, dans ledit réservoir par ledit orifice d'échappement et un pas vers le bas entre ledit orifice et une partie inférieure en contact avec ledit tampon ;
moyennant quoi, lors de l'utilisation, un liquide accumulé dans ledit réservoir peut s'écouler dans ladite bande sous l'effet de la capillarité, de la gravité et des forces de siphonnement.

2. Dispositif de test selon la revendication 1, **caractérisé en ce que** ladite bande est maintenue en une orientation non horizontale.

3. Dispositif de test selon l'une des revendications précédentes, **caractérisé en ce que** ladite bande comprend une couche unique de matériau de matrice poreux distribué uniformément.

4. Dispositif de test selon l'une des revendications précédentes, **caractérisé en ce que** ladite solution comprend un conjugué spécifique d'un état à tester.

5. Dispositif de test selon l'une des revendications précédentes, qui comprend en outre des moyens de déclenchement de la distribution dudit apport dans ladite première chambre.

6. Dispositif de test selon l'une des revendications précédentes, qui comprend en outre un puits d'échantillonnage en communication avec ladite première chambre et ayant une paroi interne en forme d'entonnoir.

7. Dispositif de test selon l'une des revendications précédentes, qui comprend en outre un tampon absorbant en contact avec une partie de ladite bande.

8. Dispositif de test selon l'une des revendications précédentes, **caractérisé en ce que** ledit apport de la solution tampon est ajusté pour créer un débordement mineur dudit réservoir.
